# EUROPÄISCHE PATENTSCHRIFT

(11) **EP 0 688 231 B1**
(45) Veröffentlichungstag und Bekanntmachung des Hinweises auf die Patenterteilung: **17.09.1997**
(21) Anmeldenummer: 93906522.3
(22) Anmeldetag: 13.03.1993
(51) Int. Cl.: A61L 9/04, A61L 9/01

(54) **RIESELFÄHIGES MITTEL ZUR LUFTVERBESSERUNG**
POURABLE AIR-FRESHENING AGENT
AGENT COULANT POUR DESODORISER L'AIR

(43) Veröffentlichungstag der Anmeldung: 27.12.1995
(73) Patentinhaber: SCHMITZ-GÖBBELS, Sabine, D-51143 Köln (DE)
(72) Erfinder: SCHMITZ-GÖBBELS, Sabine, D-51143 Köln (DE)
(74) Vertreter: Müller-Gerbes, Margot, Dipl.-Ing.
(86) Internationale Anmeldenummer: EP9300584
(87) Internationale Veröffentlichungsnummer: WO9421305

(56) Entgegenhaltungen:
- EP-A- 0 097 095
- DE-A- 4 141 097
- GB-A- 2 217 603

## Beschreibung

Die Erfindung bezieht sich auf ein rieselfähiges Mittel zur Luftverbesserung, enthaltend mineralische Silikate und Carbonate und mindestens einen Riechstoff.

Es ist bekannt, die Luft in geschlossenen Räumen, wie Toiletten, zum Beispiel mittels Lufttabletten, Duftkissen, Duftpatronen oder dergleichen zu verbessern. Hierbei handelt es sich beispielsweise um parfümierte, d.h. mit Parfumöl oder dergleichen getränkte Körper, wie Schwämme oder dergleichen. Es ist auch bekannt, in schlecht riechende Luft erzeugende Geräte, wie Staubsauger, Duftpatronen einzubauen, die an einer Stelle innerhalb des Staubsaugergehäuses angeordnet sind und von hier aus duften. Derartige Duftpatronen sind beispielsweise in der DE-OS 23 41 938 beschrieben, bei denen ein fester geformter Körper aus Magnesiumcarbonat oder Kalziumcarbonat oder Kalk mit einer Duftölessenz getränkt wird. Aus der EP 0286 601 A1 ist ein deodorierendes Pulver, enthaltend doppelt kohlensaures Natron, für Schuhe bekannt.

Des weiteren ist es auch bekannt, mit Sand gefüllten Aschenbechern zur Luftverbesserung Riechstoffe zuzugeben. Aus der GB 2217603A sind auch mineralische Mischungen aus Granulaten mit groben Körnern von Korndurchmessern von 0,5 bis 5 mm von Silikaten und Riechstoffen als Aschenbechersand bekannt, denen flammhemmende Zusätze auf Basis von Carbonaten in großen Mengen von 20 bis 70 Gew.-%, bezogen auf die Silikate, zugegeben werden können.

Der Erfindung liegt die Aufgabe zugrunde, ein Mittel zur Luftverbesserung insbesondere in geschlossenen Räumen zu schaffen, das vielseitig einsetzbar ist, das leicht nach Bedarf dosierbar ist, das problemlos wieder entfernbar ist.
Zur Lösung dieser Aufgabe schlägt die Erfindung vor, daß als Trägersubstanz für die Riechstoffe eine pulverige Trägersubstanz auf Basis von neutralen Carbonaten mit einem Korndurchmesser kleiner 0,1 mm vorgesehen ist und die Trägersubstanz mit den Riechstoffen getränkt ist und die Trägersubstanz 0,1 bis 10 Gew.-% Riechstoffe enthält, und ein Gemisch aus 3 bis 20 Gew.-% mit den Riechstoffen getränkter Trägersubstanz und 80 bis 97 Gew.-% feinkörnigem Sand hergestellt ist.
Vorteilhafte Ausgestaltungen der Erfindung sind den kennzeichnenden Merkmalen der Ansprüche 2 bis 9 entnehmbar.
Dieses erfindungsgemäße rieselfähige Gemisch kann in jedes entsprechende Gefäß, wie zum Beispiel Aschenbecher, Blumentöpfe, Schalen, eingefüllt, in beliebigen Mengen dosiert und jederzeit wieder durch Entleerung der Gefäße entfernt werden. Das erfindungsgemäße rieselfähige Gemisch kann in Dosen, Flaschen aufbewahrt werden und fein dosiert über Streulöcher entnommen werden, da es nicht verklumpt und feinkörnig ist.
Eine besonders bevorzugte Anwendung des erfindungsgemäßen Mittels ist bei der Reinigung von Räumen mittels Staubsaugern vorgesehen, um deren Abluft zu verbessern, d.h. den muffigen Staubsaugergeruch von Staubsaugerabluft aufzufrischen und zu beduften.
Es ist nun möglich, das rieselfähige erfindungsgemäße Gemisch mit jeder gewünschten Duftnote auszustatten. Wenn bei einem Reinigungsvorgang der Staubsauger mit einer neuen Tüte, Beutel ausgestattet wird, wird zugleich etwas rieselfähiges parfümiertes Gemisch auf den Boden gestreut und zuerst in die leere Staubsaugertüte aufgesaugt. Das Gemisch bleibt dann fein verteilt in der Staubsaugertüte hängen. Damit ist der Staubsauger präpariert. Alle weitere durch den Staubsauger strömende Abluft wird nun beim Vorbeistreichen an der Tüte mit den Riechstoffen aus dem hier abgelagerten Mittel angereichert und tritt mit einer entsprechenden angenehmen Duftnote beladen aus dem Staubsauger aus und erfüllt den Raum mit angenehm riechender Abluft. Da das rieselfähige parfümierte Gemisch in dem Gehäuse des Staubsaugers eingeschlossen ist, hält es sich dort über einen längeren Zeitraum, bis der Beutel voller Staub ist und durch einen neuen ersetzt wird. Sobald ein neuer Beutel oder Tüte wieder in das Staubsaugergehäuse eingesetzt ist, beginnt die Prozedur mit dem Einbringen des Mittels in die Staubsaugertüte erneut. Es ist für jeden Vorgang nur eine sehr kleine Dosis von wenigen Gramm erfindungsgemäßen Gemisches erforderlich.

Als Riechstoffe kommen insbesondere die zur Herstellung von Parfums, zur Parfümierung von Seifen, Kosmetika oder anderen Industrieprodukten benutzten Riechstoffe infrage. Hierbei unterscheidet man natürliche, halbsynthetische und synthetische Riechstoffe. Die natürlichen Riechstoffe sind die aus Pflanzen oder Pflanzenteilen isolierten ätherischen Öle, Harze und Balsame sowie tierische Sekrete, zum Beispiel Ambra, Moschus, Zibet, und daraus isolierte Einzelverbindungen oder einheitliche Fraktionen. Als halbsynthetische Riechstoffe bezeichnet man chemisch abgewandelte natürliche Riechstoffe, zum Beispiel Linalylacetat aus Linalool oder Jonone aus Citral.
Synthetische Riechstoffe gewinnt man aus einfachen organischen Rohstoffen.

Bevorzugt werden ätherische Öle als Riechstoffe eingesetzt, das sind flüchtige stark riechende ölige Produkte, die durch Wasserdampfdestillation von Pflanzen oder Pflanzenteilen oder durch Auspressen der äußeren Fruchtschalen von Citrusarten gewonnen werden. Ätherische Öle verdunsten vollständig und hinterlassen auf Papier keinen Fettfleck. Die ätherischen Öle, so wie sie natürlich gewonnen werden, enthalten überwiegend Terpene und Sesquiterpene. Die duftenden Komponenten sind die Sauerstoffderivate, wie Alkohole, Äther, Aldehyde, Ketone, Ester, Laktone, Epoxyde, aber auch einige N- und S-haltige Verbindungen. Nach der Deterpenierung, d.h. Abtrennung der geruchlosen und in Ethanol unlöslichen Terpenkohlenwasserstoffe können die ätherischen Öle zur Komposition von Parfums und zur Parfürmierung von Produkten, zur Herstellung von Essenzen und auch zur Isolierung reiner Riechstoffe eingesetzt werden. Für die Erfindung können sowohl die reinen Riechstoffe eingesetzt werden als auch bevorzugt die deterpenierten ätherischen Öle, die auch schlechthin als Parfumöle bezeichnet werden. Bevorzugt werden die Riechstoffe in konzentrierter Form eingesetzt, d.h. ohne Zusatz von Alkohol oder anderen Verdünnungsmitteln. Die Riechstoffe können in beliebigen gewünschten Duftnoten zusammengestellt werden und entsprechende Riechstoffgemische vorgesehen werden.

Erfindungsgemäß sind die Riechstoffe einer pulverigen Trägersubstanz beigegeben und diese parfümierte Trägersubstanz wird zusammen mit feinkörnigem Sand als Mittel zur Luftverbesserung verwendet. Mit den Riechstoffen wird praktisch die Trägersubstanz parfümiert, wobei die Riechstoffe durch Absorption oder Anhaftung oder Aufsaugung mit der Trägersubstanz so weit verbunden sind, daß diese sie transportiert. Als Trägersubstanz werden pulverige inerte ungiftige unbrennbare schwer wasserlösliche bis wasserunlösliche und die Riechstoffe bevorzugt durch Adhäsion aufnehmende Trägersubstanzen vorgesehen. Diese pulverigen parfümierten Trägersubstanzen werden dann in geeigneten Mengen dem Sand als Füllmaterial beigemischt. Als gut geeignet als Trägersubstanz haben sich die Salze der Kohlensäure erwiesen, insbesondere die neutralen Carbonate. Von diesen ist das Magnesiumcarbonat in pulveriger Form bevorzugt. Die Trägersubstanz kann mit dem Sand als Füllmaterial in gewünschten Mengenverhältnissen und Konzentrationen leicht gemischt werden, wodurch eine feine Verteilung der Riechstoffe in dem Gemisch des Luftverbesserungsmittels und Duftabgabe der Riechstoffe über einen längeren Zeitraum erreicht wird. Die Rieselfähigkeit des Mittels ermöglicht das leichte Befüllen und Entleeren von Gefäßen. Der Sand dient insbesondere dazu, eine gute Rieselfähigkeit des Mittels herzustellen, so daß es gut handhabbar ist und auch zum Zwecke der Reinigung ggf. leicht entfernbar ist. Infolge der Saugfähigkeit der Trägersubstanz bzw. des Anhaftens der Riechstoffe an der Trägersubstanz wird das Absetzen der flüssigen Riechstoffe - wie Parfumöle - aus dem Mittel am Boden von Gefäßen oder dergleichen vermieden. Die Anlagerung der Riechstoffe an die Trägersubstanz verhindert ein schnelles Verdunsten der Riechstoffe und sorgt damit für eine gleichmäßige Duftabgabe über einen längeren Zeitraum. Auch Kalziumcarbonat oder das natürlich vorkommende Dolomitsteinmehl eignen sich gut als Trägersubstanz. Insbesondere ist vorgesehen, die Trägersubstanz in feinster Kornform einzusetzen.

Nach einer bevorzugten Ausführung der Erfindung ist die Trägersubstanz, um Verwechselungen mit anderen Materialien, wie zum Beispiel Zucker oder Kochsalz, zu vermeiden, eingefärbt, insbesondere in den Farben braun oder beige. Es ist auch möglich, poppige Farben, wie pink, zu verwenden.

Das Mittel zur Luftverbesserung enthält als Füllmaterial feinkörnigen Sand ausgewählter Korngrößen. Um eine ausreichende Duftentwicklung zum Unterdrücken von Geruch von Tabak oder Staubluft zu erreichen, ist vorgesehen, daß die Trägersubstanz 0,1 bis 10 Gew.-%, vorzugsweise 0,3 bis 5 Gew.-%, Riechstoffe enthält.

Eine bevorzugte Zusammensetzung eines rieselfähigen Gemisches als Mittel zur Luftverbesserung enthält 85 bis 95 Gew.-% Sand und 5 bis 15 Gew.-% Trägersubstanz mit einem 0,3 bis 3 %igen Parfümölgehalt.

Nachfolgend wird ein Ausführungsbeispiel der Erfindung angegeben:
7 Gew.-Teile braun gefärbtes feinpulveriges Magnesiumcarbonat werden mit 1 Gew.-Teil eines konzentrierten Parfumöls, beispielsweise auf der Basis eines zitronenartigen oder frischblumigen Duftes, getränkt. Danach werden 90 Gew.-Teile feinkörniger sauberer Sand hinzugegeben und mit dem parfümierten Magnesiumcarbonat vermischt. Der Sand sollte trocken sein.

Das so erhaltene rieselfähige parfümierte Mittel zur Luftverbesserung kann zum Beispiel in Dosen, Flaschen oder Säcken luftdicht abgefüllt und aufbewahrt werden. Aus diesen Behältnissen kann es dann bei Bedarf entnommen werden. Da es rieselfähig ist, ist es leicht zu handhaben.

## Patentansprüche

1. Rieselfähiges Mittel zur Luftverbesserung, enthaltend mineralische Silikate und Carbonate und mindestens einen Riechstoff, **dadurch gekennzeichnet**, daß als Trägersubstanz für die Riechstoffe eine pulverige Trägersubstanz auf Basis von neutralen Carbonaten mit einem Korndurchmesser kleiner 0,1 mm vorgesehen ist und die Trägersubstanz mit den Riechstoffen getränkt ist und die Trägersubstanz 0,1 bis 10 Gew.-% Riechstoffe enthält, und ein Gemisch aus 3 bis 20 Gew.-% mit den Riechstoffen getränkter Trägersubstanz und 80 bis 97 Gew.-% feinkörnigem Sand hergestellt ist.

2. Mittel nach Anspruch 1,
**dadurch gekennzeichnet**, daß als Trägersubstanz Magnesiumcarbonat vorgesehen ist.

3. Mittel nach Anspruch 1 oder 2,
**dadurch gekennzeichnet**, daß als Trägersubstanz Kalziumcarbonat vorgesehen ist.

4. Mittel nach einem der Ansprüche 1 bis 3,
**dadurch gekennzeichnet**, daß Dolomitsteinmehl als Trägersubstanz vorgesehen ist.

5. Mittel nach einem der Ansprüche 1 bis 4,
**dadurch gekennzeichnet**, daß die Trägersubstanz 0,3 bis 5 Gew.-%, Riechstoffe enthält.

6. Mittel nach einem der Ansprüche 1 bis 5,
**dadurch gekennzeichnet**, daß die Trägersubstanz eingefärbt ist, insbesondere in brauner oder beiger Farbe.

7. Mittel nach einem der Ansprüche 1 bis 6,
**dadurch gekennzeichnet**, daß die Riechstoffe in konzentrierter Form eingesetzt sind und als Riechstoffe natürliche Riechstoffe, wie ätherische Öle, Harze, Balsame sowie tierische Sekrete, wie Ambra, Moschus, vorgesehen sind.

8. Mittel nach einem der Ansprüche 1 bis 7,
**dadurch gekennzeichnet**, daß mehr als 50 Gew.-%, insbesondere mehr als 80 Gew.-%, feinkörniger Sand mit Korndurchmessern kleiner 1,0 mm, bevorzugt kleiner 0,5 mm, eingesetzt ist.

9. Mittel nach einem der Ansprüche 1 bis 8,
**dadurch gekennzeichnet**, daß die Trägersubstanz einen Korndurchmesser kleiner 0,05 mm, aufweist.

## Claims

1. Pourable air-freshening agent comprising mineral silicates and carbonates and at least one perfuming ingredient, characterized in that there is provided as carrier for the perfuming ingredient a powder carrier based on neutral carbonates having a grain diameter below 0.1 mm and in that said carrier is soaked in perfuming ingredients and contains 0.1 to 10% by weight of perfuming ingredients, and in that a mixture of 3 to 20% by weight of perfuming ingredient soaked carrier and of 80 to 97% by weight of fine grain sand is produced.

2. Agent according to claim 1, characterized in that there is provided as carrier magnesium carbonate.

3. Agent according to claim 1 or 2, characterized in that there is provided as carrier calcium carbonate.

4. Agent according to any one of claims 1 to 3, characterized in that there is provided as carrier dolomite brick flour.

5. Agent according to any one of claims 1 to 4, characterized in that said carrier comprises 0.3 to 5% by height of perfuming ingredients.

6. Agent according to any one of claims 1 to 5, characterized in that said carrier is coloured, in particular brown or beige.

7. Agent according to any one of claims 1 to 6, characterized in that said perfuming ingredients are used in a concentrated form and in that there are used perfuming ingredients of natural origin, such as essential oils, resins, balms, as well as animal secretions such as amber or musk.

8. Agent according to any one of claims 1 to 7, characterized in that there is used more than 50% by weight, in particular more than 80% by weight, of fine grain sand having grain diameters below 1.0 mm, preferably below 0.5 mm.

9. Agent according to any one of claims 1 to 8, characterized in that the grain diameter of the carrier is below 0.05 mm.

## Revendications

1. Agent coulant pour désodoriser l'air, comprenant des silicates et carbonates minéraux et au moins un ingrédient parfumant, caractérisé en ce que l'on prévoit comme support de l'ingrédient parfumant un support sous forme d'une poudre à base de carbonates neutres ayant un diamètre de grain inférieur à 0,1 mm, et en ce que le support est imbibé d'ingrédients parfumants et contient 0,1 à 10% en poids d'ingrédients parfumants, et en ce que l'on produit un mélange de 3 à 20% en poids du support imbibé d'ingrédients parfumants et de 80 à 97% de sable à grains très fins.

2. Agent selon la revendication 1, caractérisé en ce que l'on prévoit comme support le carbonate de magnésium.

3. Agent selon la revendication 1 ou 2, caractérisé en ce que l'on prévoit comme support le carbonate de calcium.

4. Agent selon l'une des revendications 1 à 3, caractérisé en ce que l'on prévoit comme support la farine de brique de dolomie.

5. Agent selon l'une des revendications 1 à 4, caractérisé en ce que le support contient 0,3 à 5% en poids d'ingrédients parfumants.

6. Agent selon l'une des revendications 1 à 5, caractérisé en ce que le support est coloré, en particulier on couleur brune ou beige.

7. Agent selon l'une des revendications 1 à 6, caractérisé en ce que les ingrédients parfumants sont employés sous forme concentrée, et en ce que l'on prévoit des ingrédients d'origine naturelle, tels que les huiles essentielles, les résines, le baume, ainsi que des sécrétions animales, telles que l'ambre ou le musc.

8. Agent selon l'une des revendications 1 à 7, caractérisé en ce que l'on emploie plus de 50%, en particulier plus de 80% en poids, de sable à grains très fins présentant des diamètres de grain inférieurs à 1,0 mm, de préférence inférieurs à 0,5 mm.

9. Agent selon l'une des revendications 1 à 8, caractérisé en ce que le support présente un diamètre de grain inférieur à 0,05 mm.
